# EUROPÄISCHE PATENTSCHRIFT

(11) **EP 0 991 616 B1**
(45) Veröffentlichungstag und Bekanntmachung des Hinweises auf die Patenterteilung: **21.05.2003**
(21) Anmeldenummer: 98933586.4
(22) Anmeldetag: 03.06.1998
(51) Int. Cl.: C07C 51/09, A61K 9/00, A61K 47/00

(54) **VERFAHREN ZUR HERSTELLUNG VON ARZNEIMITTELN, ENTHALTEND HMG-CoA- REDUKTASEINHIBITOREN**
METHOD FOR PRODUCING MEDICAMENTS CONTAINING HMG-CoA-REDUCTASE INHIBITORS
PROCEDE POUR LA FABRICATION DE MEDICAMENTS CONTENANT DES INHIBITEURS DE LA HMG-CoA-REDUCTASE

(30) Priorität: 16.06.1997 DE 19725391
(43) Veröffentlichungstag der Anmeldung: 12.04.2000
(73) Patentinhaber: Bayer Aktiengesellschaft, 51368 Leverkusen (DE)
(72) Erfinder: SCHÜCKLER, Fritz, D-51373 Leverkusen (DE); GEISEN, Karl, D-50937 Köln (DE); PÖLLINGER, Norbert, D-79379 Müllheim (DE); SAMAAN, Samir, D-42113 Wuppertal (DE)
(86) Internationale Anmeldenummer: EP9803294
(87) Internationale Veröffentlichungsnummer: WO98057917

(56) Entgegenhaltungen:
- EP-A- 0 547 000
- WO-A-94/16693

## Beschreibung

Die Erfindung betrifft ein Verfahren zur Herstellung von Arzneimitteln, enthaltend HMG-CoA-Reduktaseinhibitoren, insbesondere in Form von Granulaten, Tabletten und Pellets.

Es ist bekannt, daß HMG-CoA-Reduktaseinhibitoren als Wirkstoffe in Arzneimitteln zur Behandlung von Hyperlipoproteinämie und Arteriosklerose eingesetzt werden. Die meisten dieser Wirkstoffe stammen aus der Reihe der Statine folgender Formel 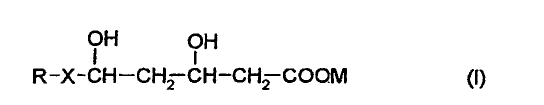 worin
- R: für einen organischen Rest steht,
- X: für eine Gruppe -CH₂-CH₂- oder -CH=CH-; insbesondere in der (E)-Form steht und
- M: ein physiologisch akzeptables Kation bedeutet, beispielsweise aus der Reihe der Alkalikationen, bevorzugt Natrium oder Kalium sowie für ein Ammonium- ion steht.

Außerdem ist bekannt, daß die meisten Wirkstoffe der Statinreihe in Form ihrer Salze eingesetzt werden. Hierzu werden diese Wirkstoffe in der Regel in wäßriger Lösung zunächst aus den entsprechenden Estern oder Säuren als Vorstufe durch Behandlung mit Basen hergestellt und diese Lösung anschließend zur Gewinnung des eigentlichen Wirkstoffes lyophilisiert (EP 547 000).

Dieses Verfahren ist naturgemäß sehr zeit- und kostenintensiv und bedingt u.a. umfangreichen Aufwand bezüglich Prozeßüberwachung, -steuerung und -optimierung. Darüber hinaus ist ein Lyophilisationsprodukt sehr schwierig zu handhaben, da es sehr stark hygroskopische Eigenschaften aufweist. Dies kann, auch trotz sorgfältiger Einhaltung von klimatisch günstigen Bedingungen (niedrige Luftfeuchtigkeit) oder besonderen feuchtigkeitsdichten Packmitteln zu beachtlichen Problemen bei der Lagerung und Herstellung führen (z.B. Ungenauigkeit bei der Einwaage, Gehaltsabweichungen in den Tabletten durch Feuchtigkeitsaufnahme im Granulat).

Es wurde nun ein Verfahren zur Herstellung von Arzneimitteln enthaltend HMG-CoA-Reduktaseinhibitoren der Statinreihe der allgemeinen Formel (I) 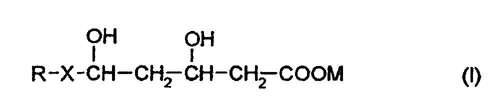 worin
- R: für einen organischen Rest steht,
- X: für eine Gruppe -CH₂-CH₂- oder -CH=CH steht und
- M: für ein pharmakologisch akzeptables Kation steht,
gefunden, dadurch gekennzeichnet; daß man aus der entsprechenden Wirkstoffvorstufe zunächst durch Behandeln mit wäßriger Base den eigentlichen Wirkstoff in wäßriger Lösung/Suspension herstellt und entweder diese wirkstoffhaltige Lösung/Suspension anschließend unmittelbar auf Hilfsstoffe aufsprüht und parallel dazu trocknet oder nach Vermischen der wirkstoffhaltigen Lösung/Suspension mit geeigneten Bindemitteln und Hilfsstoffen granuliert und anschließend trocknet.

Das erfindungsgemäße Verfahren beschreibt damit die Herstellung von Arzneimitteln, ohne daß der Wirkstoff in seine feste Form bzw. als Reinsubstanz isoliert wird, sondern als Lösung direkt weiterverarbeitet wird.

Der gesamte Herstellprozeß wird damit erheblich vereinfacht, insbesondere weil der problematische und teure Lyophilisationsschritt zur Isolierung des Wirkstoffes entfällt. Dies bedingt auch eine deutliche Verbesserung der Handhabbarkeit und Herstellsicherheit der Arzneizubereitung.

Als Basen werden im allgemeinen solche Basen eingesetzt, die Alkali- und Erdalkali-Ionen enthalten. Bevorzugt sind Hydroxide, Carbonate oder Hydrogencarbonate, die Alkali- oder Erdalkali-Ionen wie z.B. Natrium-, Kalium-, Lithium-, Berrylium-, Calcium- oder Magnesium-Ionen enthalten. Besonders bevorzugt werden Natriumhydroxid oder Kaliumhydroxid eingesetzt, abhängig davon, ob es sich bei dem eigentlichen Wirkstoff um ein Natrium- oder ein Kaliumsalz handelt.

Die Menge an Base beträgt mindestens äquimolare Menge, bezogen auf die Einsatzmenge der Wirkstoffvorstufe.

Als Vorstufen sind hierbei die entsprechenden Säuren oder Ester der Formel (II) 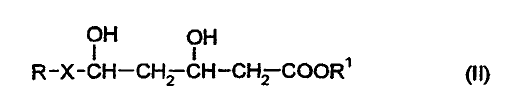 oder die entsprechenden Lactone der Formel (III) 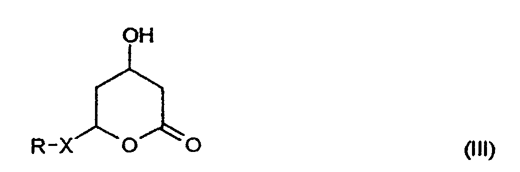 in welchen
- R: einen organischen Rest bedeutet,
- X: eine Gruppe der Formel -CH₂-CH₂- oder -CH=CH- bedeutet und
- R¹: für eine C₁-C₄-Alkylgruppe oder Wasserstoff steht
zu verstehen.

Bevorzugt werden als Vorstufen die Lactone der Formel (III) eingesetzt, wobei die eigentlichen Wirkstoffe durch Spaltung des Lactonringes durch die Base unmittelbar in der Lösung hergestellt werden.

Besonders bevorzugt eignet sich Cerivastatin-Lacton, das mit Natriumhydroxid zum Wirkstoff Cerivastatin in Lösung umgesetzt wird.

Das erfindungsgemäße Verfahren ist geeignet zur Herstellung von festen und flüssigen, insbesondere von festen Arzneiformen wie beispielsweise wirkstoffhaltigen Pulvern, Granulaten, Tabletten oder Pellets. Hierbei ist es möglich, die Pulver, Granulate oder Pellets zu Tabletten zu pressen, oder in Kapseln einzufüllen.

Als Bindemittel für die Granulation werden alle üblichen, pharmazeutisch annehmbaren Bindemittel eingesetzt, z.B. Polyvinylpyrrolidone, Gelatine, Stärke- und Cellulosederivate (natürliche bzw. synthetische).

Bevorzugt sind Polyvinylpyrrolidone, z.B. Polyvinylpyrrolidon 25.

Als weitere Hilfsstoffe können alle üblichen pharmazeutischen Hilfsstoffe eingesetzt werden, so z.B. als Füllstoffe Cellulosederivate (z.B. mikrikristalline Cellulose), Zucker (z.B. Lactose), Zuckeralkohole (z.B. Mannit, Sorbit), anorganische Füllstoffe (z.B. Calciumphosphate) sowie alle weiteren Hilfsstoffe, die zur Herstellung von Arzneiformulierungen der gewünschten Eigenschaften benötigt werden, z.B. Schmiermittel (z.B. Magnesiumstearat), z.B. Sprengmittel (z.B. quervernetztes Polyvinylpyrrolidon, Natriumcarboxymethylcellulose), z.B. Netzmittel (z.B. Natriumlaurylsulfat), z.B. Stabilisatoren, z.B. Aromen, z.B. Farbpigmente.

Bevorzugt als Füllstoffe werden Lactose, Mannitol und mikrokristalline Cellulose verwendet.

Der Anteil an Bindemittel in der Gesamtmischung ist bevorzugt 0 bis 20 %. Der Anteil an Füll- und Hilfsstoffen in der Gesamtmischung beträgt bevorzugt 70 bis 99%.

Die Trockentemperatur beträgt im allgemeinen 40 bis 120°C, bevorzugt 60 bis 100°C.

Das erfindungsgemäße Verfahren eignet sich besonders für Wirkstoffe der Formel (I), in welcher der Substituent R einen gegebenenfalls substituierten Pyrimidin-, Indol-, Indolizin-, Pyrrolo-Pyridin-, Chinolin-, Dihydrochinolin-, Pyrazolo-Pyridin-, Pyridazin-, Imidazol-, Pyrrolo-Isochinolin-, Pyridin-, Pyrrol-, Tetrazol-Rest bedeutet.

Das erfindungsgemäße Verfahren ist besonders geeignet für folgende Wirkstoffe der Formel (I):
Pravastatin,
3R,5S-(E)-7-[4-(4-fluorophenyl)-6-(1-methylethyl)-2-dimethylaminopyrimidin-5-yl]-3,5-dihydroxy-6-hepten-säure Natriumsalz;
Erythro-(±)-(E)-7-[3-(4-fluorphenyl)-spiro[cyclopentane-1,1'-1H-inden]-2'-yl]-3,5-dihydroxy-6-hepten-säure Natriumsalz;
3R,5S-(E)-7-[3-(4-flurophenyl)-1-(1-methylethyl)-indolizin-3yl]-3,5-dihydroxy-6-hepten-säure Natriumsalz;
3R,5S-(E)-7-[3-(4-fluorophenyl)-1-(1-methylethyl)-1H-pyrrolo[2,3-b]pyridin-2-yl]-3,5-dihydroxy-8- hepten-säure Natriumsalz;
3R,5S-(E)-7-[4-(4-fluorophenyl)-2-(1-methylethyl)-chinolin-3-yl]-3,5-dihydroxy-6-hepten-säure Natriumsalz;
3R,5S-(E)-7-[1-(4-fluorophenyl)-3-(1-methylethyl)-4-oxo-1,4-dihydrochinolin-2-yl]-3,5-dihydroxy-6-hepten-säure Natriumsalz;
3R,5S-(E)-7-[4-(4-fluorophenyl)-6-(1-methylethyl)-3-methyl-1H-pyrazolo[3,4-b]pyridin-5-yl]-3,5-dihydroxy-6-hepten-säure Natriumsalz;
3R,5S-(E)-7-(3-(1-methylethyl)-5,6-diphenyl-pyridazin-4-yl]-3,5-dihydroxy-6-hepten-säure Natriumsalz;
3R,5S-(E)-7-[4-(4-fluorophenyl)-6-(1-methylethyl)-2-phenyl-Pyrimidin-5-yl]-3,5-dihydroxy-6-hepten-säure Natriumsalz;
3R,5S-(E)-7-[4-(4-fluorophenyl)-1-(1-methylethyl)-3-phenyl-2-oxo-2,3-dihydroimidazol-5-yl]-3,5-dihydroxy-6- hepten-säure Natriumsalz;
3R,5S-(E)-7-[4-(4-fluorophenyl)-2-(1-methylethyl)-1-oxo-1,2-dihydro-chinolin-3-yl]-3,5-dihydroxy-6- hepten-säure Natriumsalz;
Erythro-(±)-(E)-7-[4-(4-fluorophenyl)-2-(1-methylethyl)-chinolin-3-yl]-3,5-dihydroxy-6- hepten-säure Natriumsalz;
Erythro-(±)-(E)-7-[1-(4-fluorophenyl)-3-(1-methylethyl)-pyrrolo-[2,1-a]isochinolin-2-yl]-3,5-dihydroxy-6- hepten-säure Natriumsalz;
Erythro-(±)-(E)-7-[4-cyclopropyl-6-(4-fluorophenyl)-2-(4-methoxyphenyl)pyrimidin-5-yl]-3,5-dihydroxy-6- hepten-säure Natriumsalz;
3R,5S-(E)-7-[4-(4-fluorophenyl)-2,6-dimethylpyrimidin-5-yl]-3,5-dihydroxy-8-hepten-säure Natriumsalz;
3R,5S-(E)-7-[4-(4-fluorophenyl)-6-methyl-2-phenyl-pyrimidin-5-yl]-3,5-dihydroxy-8-hepten-säure Natriumsalz;
3R,5S-(E)-7-[4-(3,5-dimethylphenyl)-6-methyl-2-phenyl-pyrimidin-5-yl]-3,5-dihydroxy-6- hepten-säure Natriumsalz;
Erythro-(±)-(E)-7-[3,4-bis(4-fluorophenyl)-6-(1-methylethyl)-pyridazin-5-yl]-3,5-dihydroxy-6- hepten-säure Natriumsalz;
Erythro-(±)-(E)-7-[1-(4-fluorophenyl)-3-(1-methylethyl)-5-phenyl-1H-pyrrol-2-yl]-3,5-dihydroxy-6- hepten-säure Natriumsalz;
Erythro-(±)-(E)-9,9-bis(4-fluorophenyl)-3,5-dihydroxy-8-(1-methyl-1H-tetrazol-5-yl)-6,8-nonadien-säure Natriumsalz;
Erythro-(±)-(E)-3,5-dihydroxy-9,9-diphenyl-6,8- hepten-säure Natriumsalz;
Erythro-(±)-(E)-7-[4-(4-fluorophenyl)-1,2-bis(1-methylethyl)-3-phenylpyrrol-2-yl]-3,5-dihydroxy-6- hepten-säure Natriumsalz;
3R,5S-(E)-7-[4,5-bis(4-fluorophenyl)-2-(1-methylethyl)-1H-imidazol-1-yl]-3,5-dihydroxy-6- hepten-säure Natriumsalz;
3R,5S-(E)-7-[4-(4-Fluorophenyl)-2,6-bis(1-methylethyl)-5-methoxymethyl-pyridin-3-yl]-3,5-dihydroxy-6- hepten-säure Natriumsalz; (Cerivastatin)
Erythro-(±)-(E)-[4-(4-fluorophenyl)-2-(1-methylethyl)-6-phenyl-pyridin-3-yl]-3, 5-dihydroxy-6- hepten-säure Natriumsalz;
Erythro-(±)-(E)-[2-(4-fluorophenyl)-4,4,6,6-tetramethyl-cyclohexen-1-yl]-3,5-dihydroxy-6-hepten-säure Natriumsalz;
Erythro-(±)-(E)-7-[4-(4-fluorophenyl)-2-cyclopropyl-chinolin-3-yl]-3,5-dihydroxy-6-hepten-säure Natriumsalz; und
Erythro-(±)-(E)-7-[4-(4-fluorophenyl)-2-(1-methylethyl)-chinolin-3-yl]-3,5-dihydroxy-6- hepten-säure Natriumsalz.

Besonders geeignet ist das erfindungsgemäße Verfahren für 3R,5S-(E)-7-[4-(4-Fluorophenyl)-2,6-bis(1-methylethyl)-5-methoxymethyl-pyridin-3-yl]-3,5-dihydroxy-6- hepten-säure Natriumsalz;

Das beschriebene Verfahren ist zudem besonders geeignet, wenn der Wirkstoff in nur sehr geringen Mengen, z.B. kleiner 5 %, bevorzugt kleiner 1 % (Gewichtsanteil in der endgültigen Formulierung) eingesetzt wird. Durch Weiterverarbeitung der Wirkstofflösung bzw. -suspension zur Granulationsflüssigkeit und anschließendes Überziehen des Trägers lassen sich Arzneizubereitungen herstellen, die sich durch hervorragende Einheitlichkeit der Wirkstoffverteilung auszeichnen. Die allgemein bekannten Probleme, die sich beim konventionellen (trockenen) Mischen von Komponenten mit stark unterschiedlichen Anteilen in einer Gesamtmischung ergeben, werden so auf einfache Weise vermieden.

### Beispiele

### Beispiel 1

In einem geeigneten Gefäß wird eine bestimmte Menge der Wirkstoffvorstufe Cerivastatin-Pyridinlacton vorgelegt. Die zur quantitativen Umsetzung zu Cerivastatin benötigte Menge Natriumhydroxid (berechnet als molares Verhältnis zwischen Cerivastatin-Pyridinlacton und Natriumhydroxid), die in Form einer wäßrigen Lösung - z.B. 2,5 % (G/G) - vorliegt sowie ein weiterer berechneter Überschuß an Wasser - z.B. die 6,6-fache Menge der Cerivastatin-Pyridinlacton-Einwaage - werden vereinigt und zu dem Feststoff gegeben. Der Ansatz wird unter geeigneten Bedingungen für eine bestimmte Zeit gehalten, wobei die Umsetzung der Vorstufe zum Wirkstoff Cerivastatin erfolgt. Die Vollständigkeit der Umsetzung wird überprüft.

Nach Beendigung der Transformation wird der Ansatz filtriert, anschließend Polyvinylpyrrolidon 25 und Wasser zugesetzt. Als Beispiel sei die folgende Zusammensetzung genannt (Angabe in [mg] bezüglich der endgültigen Formulierung):

| | |
|---|---|
| Cerivastatin | 0,1 |
| Polyvinylpyrrolidon 25 | 1,8 |
| Wasser | q.s. |

Die hierbei entstandene Granulationsflüssigkeit wird direkt auf ein geeignetes Trägermaterial, z.B. Mannitol aufgezogen, was nach derzeitigem Stand der Technik entweder in einer Mischergranulation, z.B. mit Hilfe eines Schnellmischers oder in einer Wirbelschichtgranulation erfolgen kann (Angabe in [mg] bezüglich der endgültigen Formulierung):

| | |
|---|---|
| Mannitol | 83,95 |

Nach dem Trocknen wird gesiebt und gemischt.

Das entstandene Granulat kann z.B. auch nach dem Sieben unter Zusatz von geeigneten Schmiermitteln (z.B. Magnesiumstearat) und Sprengmitteln (z.B. quervernetztes Polyvinylpyrrolidon) gemischt, zu Tabletten weiterverarbeitet (Gewicht 90 mg; Durchmesser 6 mm) und anschließend lackiert (Lichtschutz des Wirkstoffes) werden.

### Beispiel 2

Wie Beispiel 1, jedoch wird die Flüssigkeit nicht auf einen pulverförmigen Träger granuliert, sondern in geeigneten Apparaturen, z.B. Wirbelschichtgeräten mit Wurstereinsatz auf Pellets aufgezogen.

### Beispiel 3

Wie Beispiel 1, jedoch wird die Pulvergrundlage in geeigneten Apparaturen, z.B. Wirbelschichtanlagen, Rotorgranulatoren oder ähnlichen Geräten zu wirstoffhaltigen Pellets ausgerundet.

### Beispiel 4

Wie Beispiel 1, jedoch wird die Flüssigkeit in Powdercoatern auf das pulverförmige Trägermaterial aufgezogen, wobei wirkstoffüberzogene Pulver entstehen.

### Beispiel 5

Wie Beispiele 1 bis 4, jedoch werden das wirkstoffüberzogene Pulver, das Granulat bzw. die Pellets in vorgefertigte Kapseln, z.B. aus Hartgelatine oder anderen geeigneten Materialien, abgefüllt.

### Beispiel 6

Wie Beispiel 1, jedoch ist die Konzentration des Wirkstoffs pro Dosiseinheit bis auf 0,01 mg erniedrigt bzw. bis auf 5,0 mg erhöht.

### Beispiel 7

Wie Beispiel 1, jedoch wird der Granulationsflüssigkeit ein Stabilisator, z.B. Natriumhydroxid, zugesetzt.

### Beispiel 8

Wie Beispiel 1, jedoch wird der Stabilisator, z.B. Natriumcarbonat, dem Trägermaterial zugesetzt.

### Beispiel 9

Für flüssige Formulierung (wäßrige) mit Option, bei Bedarf den pH-Wert zurück zu titrieren (einzustellen, da nach Hydrolyse alkalisch).

## Patentansprüche

1. Verfahren zur Herstellung von Arzneimitteln enthaltend HMG-CoA-Reduktaseinhibitoren der Statinreihe der allgemeinen Formel I 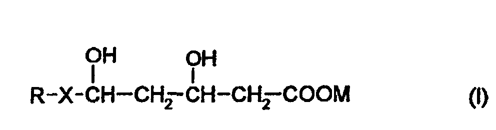 worin
R für einen organischen Rest steht,
X für eine Gruppe -CH₂-CH₂- oder -CH=CH- steht und
M für ein pharmakologisch akzeptables Kation steht,
**dadurch gekennzeichnet, daß** man durch Behandeln einer entsprechenden Wirkstoffvorstufe wobei man als Vorstufe die Säuren bzw. Ester 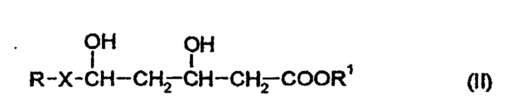 oder die entsprechenden Lactone der Formel (III einsetzt, 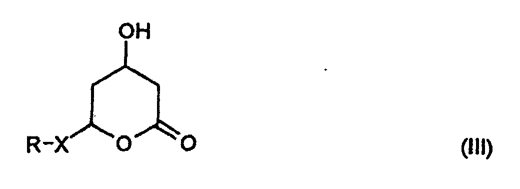 in welchen
R einen organischen Rest bedeutet,
X eine Gruppe der Formel -CH₂-CH₂- oder -CH=CH- bedeutet und
R¹ für eine C₁-C₄-Alkylgruppe oder Wasserstoff steht,
mit wäßriger Base den eigentlichen Wirkstoff in wäßriger Lösung/Suspension hersteltt und entweder diese wirkstoffhaltige Lösung/Suspension ohne Isolierung des Wirkstoffs als Festsubstanz anschließend unmittelbar auf neutrale Hilfsstoffe aufsprüht und parallel dazu trocknet oder nach Vermischen der wirkstoffhaltigen Lösung mit geeigneten Bindemitteln anschließend mit Hilfsstoffen granuliert und danach trocknet.

2. Verfahren nach Anspruch 1 , **dadurch gekennzeichnet, daß** man als Base Natriumhydroxid oder Kaliumhydroxid einsetzt.

3. Verfahren nach Anspruch 1 bis 2, **dadurch gekennzeichnet, daß** man als Bindemittel Polyvinylpyrrolidon, Gelatine, Stärke- und Cellulosederivate einsetzt.

4. Verfahren nach Anspruch 1 bis 3, **dadurch gekennzeichnet, daß** man als Hilfsstoffe Cellulosederivate, Zucker, Zuckeralkohole oder anorganische Füllstoffe.

5. Verfahren nach Anspruch 1 bis 4, **dadurch gekennzeichnet, daß** man als Hilfsstoff Mannit oder Sorbit einsetzt.

6. Verfahren nach Anspruch 1 bis 5, **dadurch gekennzeichnet, daß** der Anteil an Bindemittel in der Gesamtmischung 0-20 % beträgt.

7. Verfahren nach Anspruch 1 bis 6, **dadurch gekennzeichnet, daß** der Anteil an Füll- und Hilfsstoffen in der Gesamtmischung 70-99% beträgt.

8. Verfahren nach Anspruch 1 bis 7, **dadurch gekennzeichnet, daß** man mindestens äquimolare Menge an Base gerechnet auf die Wirkstoffvorstufe einsetzt.

9. Verfahren nach Anspruch 1 bis 8, **dadurch gekennzeichnet, daß** die Wirkstoffmenge weniger als 5 % von der Gesamtmenge der endgültigen Arzneiform beträgt.

## Claims

1. Process for producing medicaments which comprise HMG CoA reductase inhibitors of the statin group having the general formula I 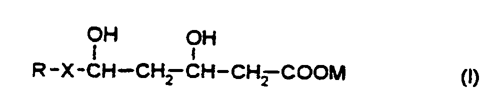 in which
R represents an organic radical,
X represents a group -CH₂-CH₂- or -CH=CH-, and
M represents a pharmacologically acceptable cation,
**characterized in that** the actual active compound is prepared in aqueous solution/suspension by treating a corresponding active compound precursor, the acids or esters 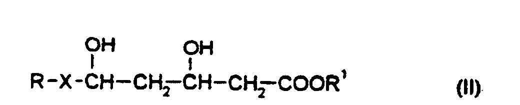 or the corresponding lactones of the formula (III) 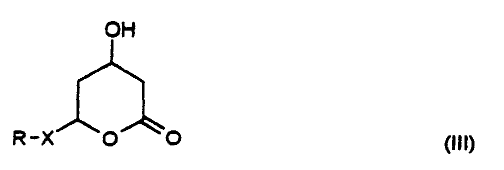 in which
R represents an organic radical,
X represents a group of the formula -CH₂-CH₂- or -CH=CH-, and
R¹ represents a C₁-C₄-alkyl group or hydrogen, being employed as precursors, with an aqueous base, and this active compound-containing solution/suspension is then either directly sprayed onto neutral auxiliary substances, without isolating the active compound as a solid substance, and dried in parallel with this, or, after mixing the active compound-containing solution with suitable binders, is then granulated with auxiliary substances and subsequently dried.

2. Process according to Claims 1, **characterized in that** sodium hydroxide or potassium hydroxide is employed as base.

3. Process according to Claims 1 and 2, **characterized in that** polyvinylpyrrolidone, gelatin or starch and cellulose derivatives is/are employed as binders.

4. Process according to Claims 1 to 3, **characterized in that** cellulose derivatives, sugars, sugar alcohols or inorganic fillers are employed as auxiliary substances.

5. Process according to Claims 1 to 4, **characterized in that** mannitol or sorbitol is employed as auxiliary substance.

6. Process according to Claims 1 to 5, **characterized in that** the proportion of binder in the total mixture is 0-20%.

7. Process according to Claims 1 to 6, **characterized in that** the proportion of fillers and auxiliary substances in the total mixture is 70-99%.

8. Process according to Claims 1 to 7, **characterized in that** at least an equimolar quantity of base, calculated in relation to the active compound precursor, is employed.

9. Process according to Claims 1 to 8, **characterized in that** the quantity of active compound is less than 5% of the total quantity of the final medicinal form.

## Revendications

1. Procédé de préparation de médicaments contenant des inhibiteurs de HMG-CoA-réductase de la série des statines de formule générale I 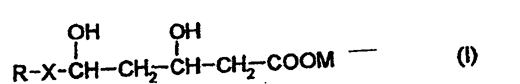 dans laquelle
R est un reste organique,
X est un groupe -CH₂-CH₂- ou -CH=CH- et
M est un cation acceptable du point de vue pharmacologique,
**caractérisé en ce que**, par traitement d'un précurseur correspondant de la substance active, en utilisant comme précurseur les acides ou esters de formule 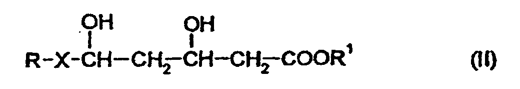 ou les lactones correspondantes de formule (III) 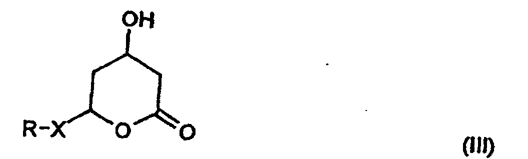 dans lesquelles
R est un reste organique,
X est un groupe de formule -CH₂-CH₂- ou -CH=CH- et
R¹ est un groupe alkyle en C₁ à C₄ ou l'hydrogène,
on prépare avec une base aqueuse la substance active concernée en solution/suspension aqueuse et ou bien on pulvérise cette solution/suspension contenant la substance active sans isoler la substance comme substance solide, directement sur des substances auxiliaires neutres et on sèche en même temps, ou bien après mélange de la solution contenant la substance active avec des liants appropriés, on granule le mélange avec des substances auxiliaires, puis on sèche.

2. Procédé suivant la revendication 1, **caractérisé en ce qu'**on utilise comme base l'hydroxyde de sodium ou l'hydroxyde de potassium.

3. Procédé suivant la revendication 1 ou 2, **caractérisé en ce qu'**on utilise comme liant la polyvinylpyrrolidone, la gélatine, des dérivés d'amidon et des dérivés de cellulose.

4. Procédé suivant les revendications 1 à 3, **caractérisé en ce qu'**on utilise comme substances auxiliaires des dérivés de cellulose, des sucres, des sucres-alcools ou des charges inorganiques.

5. Procédé suivant les revendications 1 à 4, **caractérisé en ce qu'**on utilise comme substance auxiliaire le mannitol ou le sorbitol.

6. Procédé suivant les revendications 1 à 5, **caractérisé en ce que** la proportion de liant dans le mélange total est de 0 à 20 %.

7. Procédé suivant les revendications 1 à 6, **caractérisé en ce que** la proportion de charges et de substances auxiliaires dans le mélange total est de 70 à 99 %.

8. Procédé suivant les revendications 1 à 7, **caractérisé en ce qu'**on utilise au moins une quantité équimolaire de base calculée sur le précurseur de substance active.

9. Procédé suivant les revendications 1 à 8, **caractérisé en ce que** la quantité de substance active est inférieure à 5 % de la quantité totale de la forme médicamenteuse définitive.
